# EUROPEAN PATENT SPECIFICATION

(11) **EP 4 125 755 B1**
(45) Date of publication and mention of the grant of the patent: **16.07.2025**
(21) Application number: 21714696.8
(22) Date of filing: 30.03.2021
(51) Int. Cl.: A61F 13/15, A61F 13/53, A61F 13/514, A61F 13/534

(54) **COMPOSITE WEB**
VERBUNDBAHN
BANDE COMPOSITE

(30) Priority: 01.04.2020 IT 202000006826; 01.04.2020 IT 202000006835
(43) Date of publication of application: 08.02.2023
(73) Proprietor: GDM S.P.A., 40133 Bologna (IT)
(72) Inventor: ZAVALLONI, Alessandro, 40133 Bologna (IT); PIANTONI, Matteo, 40133 Bologna (IT); GIARDINI, Francesco, 40133 Bologna (IT); ROSANI, Marco, 40133 Bologna (IT)
(74) Representative: Puggioli, Tommaso
(86) International application number: PCT/IB2021/052609
(87) International publication number: WO 2021/198894

(56) References cited:
- US-A- 4 429 001
- US-A- 4 469 734
- US-A- 4 797 318
- US-A- 5 952 251
- US-A1- 2003 106 560
- US-A1- 2004 243 080
- US-A1- 2019 021 913

## Description

### Technical field

This invention relates to a composite web and, in particular, a composite web comprising melted and blown fibres and particulate material, for example, absorbent or superabsorbent material.

### Background art

Known in the prior art are composite webs which comprise melted and blown fibres, known as "meltblown" fibres, and spunbond, in which a particulate material, for example, in the form of discrete particles, is trapped.

The particulate material may be, for example, an absorbent or superabsorbent polymer material and examples of composite webs comprising meltblown fibres and discrete particles are described and illustrated in documents US6494974 and EP0156160.

By absorbent (or superabsorbent) material is meant a material that is capable of retaining any liquid or of filtering a fluid (liquid or gas) containing an extraneous substance (solid, liquid or gas).

With regard to composite webs comprising meltblown fibres in which discrete particles of material absorbent of liquid is trapped, the need is felt to improve liquid absorption capacity for the same quantity of discrete particles of absorbent or superabsorbent material present: that is to say, to reduce the quantity of discrete particles of superabsorbent material used without reducing the amount of liquid absorbed.

US4469734 and US4797318 disclose a composite web comprising a mass of meltblown fibres comprising a melted and blown thermoplastic material and a particulate absorbent or superabsorbent material dispersed among the fibres and at least partly adherent thereto.

US4429001 discloses a composite web, comprising a mass of meltblown fibres comprising a melted and blown thermoplastic material and superabsorbent material and surfactant particles mixed together and introduced into the stream of the fibres.

### Disclosure of the invention

In this context, the intention is to propose a composite web comprising meltblown fibres, in which discrete particles, specifically of absorbent or superabsorbent material, are trapped, capable of meeting the above mentioned need.

More specifically, this invention has for an aim to provide a composite web comprising meltblown fibres and discrete particles of absorbent or superabsorbent material, in which the quantity of discrete particles dispersed and trapped in the fibres is optimized according to the expected performance of the composite web.

This aim is achieved by a composite web comprising the technical features described in one or more of the accompanying claims. The dependent claims correspond to possible different embodiments of the invention.

According to an aspect, the invention regards a composite web comprising a mass of meltblown fibres comprising a first melted and blown thermoplastic material and a particulate material dispersed among the fibres and at least partly adherent thereto.

According to an aspect of the disclosure, the first thermoplastic material is an elastomer or an elastomeric material.

In practice, the composite web is a meltblown web formed from at least one thermoplastic elastomeric material.

The composite web comprises a hydrophilic additive melted and blown together with the first thermoplastic material to make the composite web hydrophilic.

An additive to make the composite web hydrophilic can advantageously be used especially in the case where the particulate material comprises an absorbent or superabsorbent material.

In such an application, the hydrophilic property of the fibres allows the liquid that has to be absorbed by the web to effectively reach the absorbent or superabsorbent material, for example, superabsorbent polymer (SAP) dispersed among the fibres.

The elastomer makes the fibres suitably elastic, that is to say, flexible enough to follow the SAP as it absorbs and expands, while at the same time holding it within the fibre matrix and stopping it from being lost.

That way, if, for example, the composite web according to this disclosure is used to make an absorbent pad of an absorbent product for sanitary use (nappy), the integrity of the pad, amongst other things, is advantageously improved.

Moreover, thanks to the properties of the elastomer, the fibres are able to deform to a greater extent and can therefore expand freely and absorb more liquid than in the case where it is constrained; thus, less SAP is needed to absorb the same amount of liquid.

According to an aspect of the disclosure, the suitably reduced diameter of the fibres contributes to conferring adequate elasticity to follow the expansion of the SAP.

According to an aspect of the disclosure, a suitable density of fibres - that is, the number of fibres present in the composite web - makes the web more capable of following the expansion of the SAP; in effect, with less fibres distributed around them, the SAP particles are freer to expand better.

According to an aspect of the disclosure, the composite web comprises a second thermoplastic material in the fibres.

According to an aspect of the disclosure, the composite web comprise an auxiliary web coupled to the mass of meltblown fibres in which the particulate material is dispersed.

Advantageously, thanks to the elastomer present in the fibres, the auxiliary web and the mass of meltblown fibres can be coupled without using glue or adhesive. In effect, in particular during extrusion and blowing, the elastomer is sticky and allows the fibres to stick to the auxiliary web.

In the case of superabsorbent material dispersed in the fibres, the auxiliary web, generally speaking, contributes to holding together the padding and the mass of fibres incorporating the superabsorbent material.

According to an aspect, this disclosure regards an absorbent article, for example, a nappy, provided with an absorbent pad comprising a piece of a composite web made according to this disclosure.

### Brief description of the drawings

Further features and advantages of the invention are more apparent in the exemplary, hence non-limiting description which follows of a preferred but non-exclusive embodiment of a composite web comprising meltblown fibres and particulate material.

The description is set out below with reference to the accompanying drawings which are provided solely for purposes of illustration without restricting the scope of the invention and in which:
- Figure 1 shows a schematic side view of a composite web comprising meltblown fibres and particulate material dispersed therein;
- Figure 2 shows a microscope image of a composite web comprising meltblown fibres and particulate material dispersed therein, in a first operating condition;
- Figure 3 shows the composite web of Figure 1 in a second operating condition;
- Figure 4 shows a schematic plan view of an absorbent article comprising a composite web comprising meltblown fibres and particulate material dispersed therein.

### Detailed description of preferred embodiments of the invention

With reference to the accompanying drawings, the numeral 1 denotes a composite web according to this disclosure.

The web 1 is of the type known as meltblown or spunbond - that is to say, obtained by extruding and blowing thermoplastic material.

The web 1 comprises a mass or bulk or matrix 2 of meltblown fibres 3.

In a preferred embodiment, the fibres 3 are between 0.1 microns and 30 microns in diameter, preferably between 0.5 microns and 10 microns.

In a preferred embodiment, the fibres 3 are between 0.1 gsm and 300 gsm in gram weight, preferably between 1 gsm and 20 gsm.

The fibres 3 comprise at least one meltblown thermoplastic material - that is, a thermoplastic material that is placed in an extruder to make the web 1.

In a preferred embodiment, the thermoplastic material is preferably polymeric - for example, polypropylene (PP) or polyethylene (PE) is present in the fibres 3 preferably in a percentage between 70% and 95% by weight.

The thermoplastic material has a melt flow rate of between 1 and 2000, preferably 1200.

According to this invention, the fibres 3 comprise an elastomer, that is, a substance capable of reducing the elasticity of the fibres 3 in the sense of decreasing their resistance to deformation under mechanical stress.

The elastomer may be melted and blown individually (hence constituting all of the thermoplastic material), melted and blown together with a different thermoplastic material or melted and blown together with more than one thermoplastic material (that is, the elastomer is placed in the extruder to make the meltblown web 1).

Generally speaking, the thermoplastic materials are thermoplastic materials suitable for being melted and blown. Preferably, the thermoplastic material or materials may be selected, by way of non-exhaustive example, from the following group of materials: polypropylene and derivatives, polyethylene and derivatives, polylactic acid and derivatives, polyhydroxyalkanoates and derivatives, cellulose and derivatives, cellulose acetate and derivatives, starch and derivatives, polyesters (for example, polyethylene terephthalate), polyurethanes, polyamides, polycarbonates, thermoplastic biopolymers in general, polyoxymethylene and derivatives, polysulfone and derivatives, methacrylates and derivatives.

When extruded together with at least one other thermoplastic material, that is, when it itself constitutes the thermoplastic material, the elastomer serves in particular to reduce the elasticity of the fibres 3, that is, to make them more flexible and pliable.

In a preferred embodiment, the fibres 3 may, as mentioned above, be made entirely from an elastomeric thermoplastic material - for example, Vistamaxx^{™} - so as to be particularly elastic.

In other words, in a preferred embodiment, the elastomer itself constitutes the first thermoplastic material, that is to say, the mass 2 may be made substantially entirely of elastomer.

In an embodiment, the elastomer is present in the fibres in a percentage between 5% and 30% by weight and is combined with at least one other thermoplastic material.

The web 1 comprises a particulate material 4 dispersed among the fibres 3 and at least partly adherent thereto.

The particulate material is present in the composite web 1 with a gram weight of between 8 and 990 gsm, preferably between 50 and 700 gsm. The particulate material 4 comprises an absorbent or superabsorbent material, for example, SAP such as Saviva B3, intended essentially for absorbing liquids.

Advantageously, the elastomer present in the fibres 3, or constituting the fibres themselves, makes the fibres particularly sticky, especially during melt blowing.

That way, the particulate material 4 adheres better to, and is retained by, the fibres.

The gram weight and diameter of the fibres 3 at least partly determine their distribution, which contributes to retaining the particulate material.

The fibres 3 comprise a hydrophilic additive which makes the fibres hydrophilic.

The hydrophilic additive is added in the extruder or even, for example, sprayed on the mass 2 after the melt blowing process.

If the hydrophilic additive is added in the extruder, the hydrophilic additive may be water based or non-water based.

The hydrophilic additive is extruded with the thermoplastic materials and the elastomer.

The hydrophilic additive is present in the fibres 3 in a percentage between 1% and 10% by weight, preferably in a percentage between 3% and 6% by weight.

The composite web 1 as a whole has a gram weight preferably between 50 and 1500 gsm.

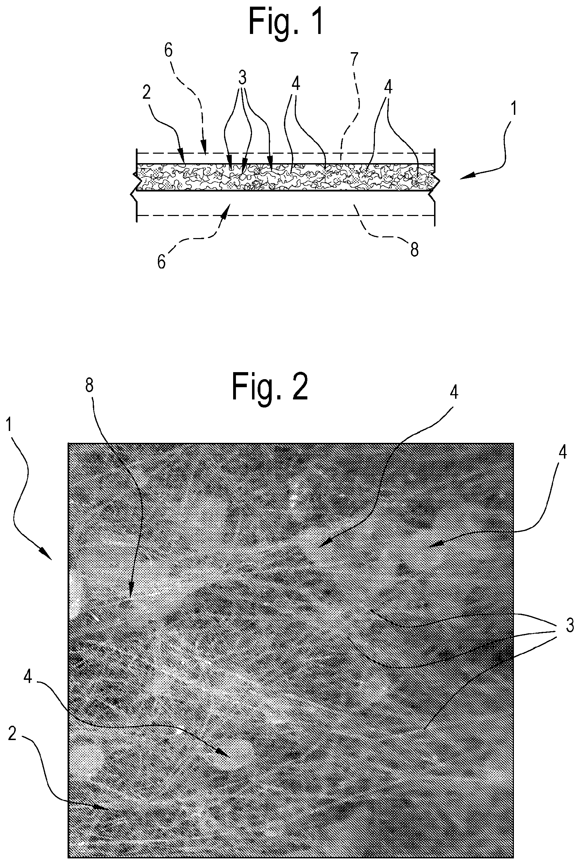

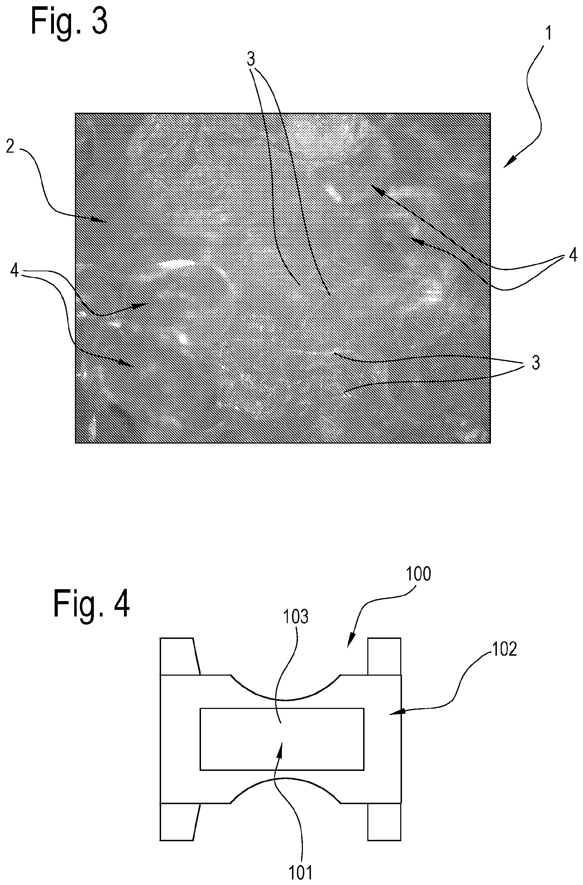

In an example embodiment, the fibres 3 comprise a polymeric material, the hydrophilic additive and the elastomer, and the particulate material comprises absorbent or superabsorbent material; the web 1 defines a composite, meltblown absorbent web illustrated, for example, in Figures 2 and 3, which show it, respectively, in a dry condition and in a wet condition, after it has absorbed a liquid.

A composite web 1 made in this way can be used, for example, to make absorbent pads intended for absorbent sanitary articles or even for food-related or medical applications.

Advantageously, as mentioned above, the fibres 3 may, in another example, be made by melt blowing only the elastomer.

The elastomer present in the fibres 3, hence their elasticity, their gram weight and their diameter contribute to holding together the absorbent material when it expands, thus optimizing what is known as *wet integrity* which may reach well over 100, compared, for example, to the values between 10 and 80 of traditional cellulose padding.

Reduced elasticity and/or fibre diameter and/or fibre density contribute to maximizing the percentage of active SAP, reduce the constraint on it, and let it expand freely without loss during absorption of the liquid.

As explained below, the reduced constraint on the SAP was confirmed by the Applicant by measuring what is known as "free absorption" and "centrifugal retention capacity" (CRC), which are higher compared to prior art solutions, thus indicating better usage of the SAP in percentage terms.

Advantageously, therefore, the absorbent capacity of the composite web 1 is improved over the same quantity of absorbent material of a traditional absorbent web.

SAP loss is also very limited, meaning that the dry integrity, at less than 1%, is much improved.

In practice, even compared to prior art solutions, SAP loss (dry integrity), SAP usage capacity and wet integrity are, in a single product, all very limited.

The additive used for making the web hydrophilic may be sprayed on the meltblown web instead of being added during extrusion. If the particulate material is a superabsorbent, such as SAP, the additive must be non-water based so as not to interact with the superabsorbent material itself.

As shown by the dashed lines in Figure 1, in a preferred embodiment, the composite web 1 may comprise at least one auxiliary web 5 and/or 6, coupled to the mass 2 of meltblown fibres in which the particulate material 4 is dispersed.

In an embodiment in which the composite web 1 comprises both auxiliary webs 5 and 6, these may form what is known as a "core cover", in particular in the case where the composite web 1 is of the absorbent type, preferably as described above.

In such a case, the webs 5 and 6 contribute to retaining the particulate material 4 and to the strength of the composite web 1.

Preferably, the auxiliary webs 5 and/or 6 comprise a web 7 of non-woven fabric - for example, meltblown or spunbond -, the web 7 of non-woven fabric having preferably a gram weight of between 1 gsm and 20 gsm.

In an embodiment, the composite web 1 comprises at least one auxiliary web 5 or 6 which comprises a web 8 of non-woven fabric, for example, of the acquisition distribution layer (ADL) or high loft type.

Preferably, the web 8 of non-woven fabric has a gram weight between 10 gsm and 300 gsm and may serve to retain the SAP, to increase the strength and softness of the composite web 1 and to distribute the liquid absorbed.

Advantageously, in an embodiment, the auxiliary web 5 and/or 6, that is, the webs of non-woven fabric 7, 8 and the mass 2 of fibres 3 are coupled without adhesive.

More specifically, the elastomer present in the fibres 3 allows joining the mass 2 to the auxiliary web 5 and/or 6.

Preferably, in such a case, when the fibres 3 are melted and blown, they are deposited directly on an auxiliary web 5 or 6 to define a supporting layer acting, for example, as a facing layer or acquisition layer in an absorbent pad comprising the composite web 1.

In an embodiment, the auxiliary web 5 and/or 6 comprises cellulose.

Described below are 3 examples of composite web according to this disclosure, preferably intended to make absorbent pads or cores.

For each example, a summary of the characteristics and relevant performance features detected by the Applicant is provided.

What is known as "usage of SAP" was estimated as the ratio, expressed as a percentage, between the centrifugal retention capacity (CRC) of the composite web and the CRC of the pure SAP and/or the ratio, expressed as a percentage, between the free absorption obtained by the composite web and the free absorption of the pure SAP.

The centrifugal retention capacity was measured according to the Edana standard method NWSP 2410R2 and the free absorption according to the Edana standard method NWSP 2400R2; the tables below show the usage of SAP calculated as web CRC/pure SAP CRC.

The tensile strength was measured with the Edana standard method 110.4.R0. The wet integrity was tested according to an in-house method, using Philip

Hardy equipment, well known in the trade, since there is no standard laboratory method for testing the integrity of an absorbent core.

In this case, each test piece was weighed and, after pouring 50 ml of sodium chloride (NaCl) on it, it was left to rest for 15 minutes; the test piece was hung in the testing equipment and breaks in the absorbent structure and the times at which they began were recorded.

The dry integrity was also tested using an in-house method of the Applicant and the loss of SAP in the dry product was measured.

A test piece or sample was cut from the composite web 1 and weighed. The test piece was shaken three times and the amount of SAP that dropped out was measured; the SAP lost was calculated as the ratio between the SAP that dropped out and the total weight of the test piece. Advantageously, the composite web according to this disclosure obtained excellent results in terms of both dry and wet integrity.

| **Example 1** | | | | |
|---|---|---|---|---|
| Ingredient | | Fibre % | | Gram weight gsm |
| Thermoplastic material | Polypropylene PP | 90 | | 9 |
| Elastomer | Vistamaxx^{™} | 5 | | |
| Hydrophilic additive | Techmer PPM15560 | 5 | | |
| Particulate material | SAP Saviva B3 | | | 92.5 |
| | | | | |

| Performance feature | Test method | | Result | |
|---|---|---|---|---|
| Usage of SAP | Edana NWSP 2410R2 | | ABS [g/g] 35.67 | |
| | Edana NWSP 2400R2 | | CRC [g/g] 22.92 | |
| | Web CRC/SAP CRC [%] | | 62% | |
| Tensile strength | Edana 110.4.RO | | >5 | |
| Wet integrity | Applicant | | >>100 | |
| Dry integrity | Applicant | | <1% | |

| **Example 2** | | | | | |
|---|---|---|---|---|---|
| Ingredient | | | Fibre % | | Gram weight gsm |
| Thermoplastic material | Polypropylene PP | | 90 | | 4.5 |
| Elastomer | Vistamaxx^{™} | | 5 | | |
| Hydrophilic additive | Techmer PPM15560 | | 5 | | |
| Particulate material | SAP Saviva B3 | | | | 44 |
| | | | | | |

| Performance feature | | Test method | | Result | |
|---|---|---|---|---|---|
| Usage of SAP | | Edana NWSP 2410R2 | | ABS [g/g] 62.75 | |
| | | Edana NWSP 2400R2 | | CRC [g/g] 27.54 | |
| | | Web CRC/SAP CRC [%] | | 75% | |
| Tensile strength | | Edana 110.4.R0 | | >5 | |
| Wet integrity | | Applicant | | >>100 | |
| Dry integrity | | Applicant | | <1% | |

| **Example 3** | | | | | |
|---|---|---|---|---|---|
| Ingredient | | | Fibre % | | Gram weight gsm |
| Thermoplastic material | Polypropylene PP | | 90 | | 4.5 |
| Elastomer | Vistamaxx^{™} | | 5 | | |
| Hydrophilic additive | Techmer PPM15560 | | 5 | | |
| Particulate material | SAP Saviva B3 | | | | 92.5 |
| Auxiliary web | Meltblown | | | | 4.5 |
| | | | | | |

| Performance feature | | Test method | | Result | |
|---|---|---|---|---|---|
| Usage of SAP | | Edana NWSP 2410R2 | | ABS [g/g] 32.64 | |
| | | Edana NWSP 2400R2 | | CRC [g/g] 24.79 | |
| | | Web CRC/SAP CRC [%] | | 67% | |
| Tensile strength | | Edana 110.4.R0 | | >5 | |
| Wet integrity | | Applicant | | >>100 | |
| Dry integrity | | Applicant | | <1% | |

With reference to Figure 4, the numeral 100 denotes an absorbent article according to this disclosure - for example, a nappy - described only insofar as necessary for understanding this invention.

The absorbent article 100 comprises an absorbent portion or pad 101 and a frame 102 on which the absorbent pad 101 is positioned.

The pad 101 comprises a piece 103 of a composite web 1 as described in the foregoing.

More specifically, the piece 103 includes at least one elastomer in the mass of fibres and superabsorbent material in the form of particulate material dispersed in the fibres.

## Claims

1. A composite web, comprising:
- a mass (2) of meltblown fibres (3) comprising at least a first, melted and blown thermoplastic material and a hydrophilic additive melted and blown together with the first thermoplastic material, the hydrophilic additive making the fibres hydrophilic; the composite web further comprising
- a particulate material (4) dispersed among the fibres and at least partly adherent thereto, wherein the first thermoplastic material is an elastomer and the particulate material (4) comprises an absorbent or superabsorbent material.

2. The composite web according to claim 1, wherein the elastomer is present in the fibres in a percentage between 5% and 30% by weight.

3. The composite web according to claim 1 or 2, wherein the hydrophilic additive is present in a percentage between 1% and 10% by weight, preferably in a percentage between 3% and 6% by weight.

4. The composite web according to any one of the preceding claims, wherein the particulate material is present with a gram weight of between 8 and 990 gsm, preferably between 50 and 700 gsm.

5. The composite web according to any one of the preceding claims, wherein the mass (2) of meltblown fibres (3) comprises a second thermoplastic material present in a percentage between 70% and 95% by weight.

6. The composite web according to claim 5, wherein the second thermoplastic material has a melt flow rate of between 1 and 2000, preferably a melt flow rate of 1200.

7. The composite web according to any one of the preceding claims, wherein the first thermoplastic material has a melt flow rate of between 1 and 2000, preferably a melt flow rate of 1200.

8. The composite web according to any one of the preceding claims, wherein the fibres are between 0.5 microns and 10 microns.

9. The composite web according to any one of the preceding claims, wherein the fibres are between 0.1 gsm and 300 gsm in gram weight, preferably between 1 gsm and 20 gsm.

10. The composite web according to any one of the preceding claims, having a gram weight of between 50 and 1500 gsm.

11. The composite web according to any one of the preceding claims, comprising at least one auxiliary web (5, 6) coupled to the mass (2) of meltblown fibres (3) in which the particulate material (4) is dispersed.

12. The composite web according to claim 11, wherein the auxiliary web (5, 6) comprises a first web (7) of non-woven fabric, the first web of non-woven fabric having preferably a gram weight of between 1 gsm and 20 gsm.

13. The composite web according to claim 11 or 12, wherein the auxiliary web (5, 6) comprises a second web (8) of non-woven fabric having a gram weight of between 10 gsm and 300 gsm.

14. The composite web according to any one of claims 11 to 13, wherein the auxiliary web (5, 6) and/or the first web (7) of non-woven fabric and/or the second web (8) of non-woven fabric and the mass (2) of meltblown fibres (3) are bonded without adhesive materials.

15. An absorbent article comprising a piece (103) of a composite web according to any one of the preceding claims.

## Patentansprüche

1. Verbundbahn, umfassend:
- eine Masse (2) aus schmelzgeblasenen Fasern (3), umfassend mindestens ein erstes geschmolzenes und geblasenes Thermoplastmaterial und ein zusammen mit dem ersten Thermoplastmaterial geschmolzenes und geblasenes hydrophiles Additiv, wobei das hydrophile Additiv dafür sorgt, dass die Fasern hydrophil werden, wobei die Verbundbahn zudem Folgendes umfasst
- ein Partikelmaterial (4), das unter den Fasern dispergiert ist und zumindest teilweise an diesen haftet, wobei das erste Thermoplastmaterial ein Elastomer ist und das Partikelmaterial (4) ein Absorber- oder Superabsorbermaterial umfasst.

2. Verbundbahn nach Anspruch 1, wobei das Elastomer in den Fasern in einem Gewichtsanteil zwischen 5 % und 30 % vorliegt.

3. Verbundbahn nach Anspruch 1 oder 2, wobei das hydrophile Additiv in einem Gewichtsanteil zwischen 1 % und 10 %, vorzugsweise in einem Gewichtsanteil zwischen 3 % und 6 %, vorliegt.

4. Verbundbahn nach einem der vorhergehenden Ansprüche, wobei das Partikelmaterial mit einem Grammgewicht zwischen 8 und 990 g/m², vorzugsweise zwischen 50 und 700 g/m², vorliegt.

5. Verbundbahn nach einem der vorhergehenden Ansprüche, wobei die Masse (2) aus schmelzgeblasenen Fasern (3) ein zweites Thermoplastmaterial umfasst, das in einem Gewichtsanteil zwischen 70 % und 95 % vorliegt.

6. Verbundbahn nach Anspruch 5, wobei das zweite Verbundmaterial einen Schmelzflussindex zwischen 1 und 2000, vorzugsweise einen Schmelzflussindex von 1200, aufweist.

7. Verbundbahn nach einem der vorhergehenden Ansprüche, wobei das erste Verbundmaterial einen Schmelzflussindex zwischen 1 und 2000, vorzugsweise einen Schmelzflussindex von 1200, aufweist.

8. Verbundbahn nach einem der vorhergehenden Ansprüche, wobei die Fasern zwischen 0,5 Mikrometern und 10 Mikrometern sind.

9. Verbundbahn nach einem der vorhergehenden Ansprüche, wobei die Fasern ein Grammgewicht zwischen 0,1 g/m² und 300 g/m², vorzugsweise zwischen 1 g/m² und 20 g/m², aufweisen.

10. Verbundbahn nach einem der vorhergehenden Ansprüche, aufweisend ein Grammgewicht zwischen 50 und 1500 g/m².

11. Verbundbahn nach einem der vorhergehenden Ansprüche, umfassend mindestens eine Hilfsbahn (5, 6), die mit der Masse (2) aus schmelzgeblasenen Fasern (3) gekoppelt ist, in der das Partikelmaterial (4) dispergiert ist.

12. Verbundbahn nach Anspruch 11, wobei die Hilfsbahn (5, 6) eine erste Bahn (7) aus Vliesstoff umfasst und die erste Bahn aus Vliesstoff vorzugsweise ein Grammgewicht zwischen 1 g/m² und 20 g/m² aufweist.

13. Verbundbahn nach Anspruch 11 oder 12, wobei die Hilfsbahn (5, 6) eine zweite Bahn (8) aus Vliesstoff umfasst, aufweisend ein Grammgewicht zwischen 10 g/m² und 300 g/m².

14. Verbundbahn nach einem der Ansprüche 11 bis 13, wobei die Hilfsbahn (5, 6) und/oder die erste Bahn (7) aus Vliesstoff und/oder die zweite Bahn (8) aus Vliesstoff und die Masse (2) aus schmelzgeblasenen Fasern (3) ohne Haftmaterialien gebunden sind.

15. Saugfähiger Artikel, umfassend ein Stück (103) einer Verbundbahn nach einem der vorhergehenden Ansprüche.

## Revendications

1. Bande composite, comprenant :
- une masse (2) de fibres fondues-soufflées (3) comprenant au moins un premier matériau thermoplastique fondu et soufflé et un additif hydrophile fondu et soufflé avec le premier matériau thermoplastique, l'additif hydrophile rendant les fibres hydrophiles ; la bande composite comprenant en outre
- un matériau particulaire (4) dispersé parmi les fibres et au moins partiellement adhérent à celles-ci, où le premier matériau thermoplastique est un élastomère et le matériau particulaire (4) comprend un matériau absorbant ou superabsorbant.

2. Bande composite selon la revendication 1, dans laquelle l'élastomère est présent dans les fibres en un pourcentage compris entre 5 % et 30 % en poids.

3. Bande composite selon la revendication 1 ou 2, dans laquelle l'additif hydrophile est présent en un pourcentage compris entre 1 % et 10 % en poids, de préférence en un pourcentage compris entre 3 % et 6 % en poids.

4. Bande composite selon l'une quelconque des revendications précédentes, dans laquelle le matériau particulaire est présent avec un poids en grammes compris entre 8 et 990 g/m², de préférence entre 50 et 700 g/m².

5. Bande composite selon l'une quelconque des revendications précédentes, dans laquelle la masse (2) de fibres fondues-soufflées (3) comprend un second matériau thermoplastique présent en un pourcentage compris entre 70 % et 95 % en poids.

6. Bande composite selon la revendication 5, dans laquelle le second matériau thermoplastique a un indice de fluidité à l'état fondu compris entre 1 et 2000, de préférence un indice de fluidité à l'état fondu de 1200.

7. Bande composite selon l'une quelconque des revendications précédentes, dans laquelle le premier matériau thermoplastique a un indice de fluidité à l'état fondu compris entre 1 et 2000, de préférence un indice de fluidité à l'état fondu de 1200.

8. Bande composite selon l'une quelconque des revendications précédentes, dans laquelle les fibres se situent entre 0,5 micron et 10 microns.

9. Bande composite selon l'une quelconque des revendications précédentes, dans laquelle les fibres ont un poids en grammes compris entre 0,1 g/m² et 300 g/m², de préférence entre 1 g/m² et 20 g/m².

10. Bande composite selon l'une quelconque des revendications précédentes, ayant un poids en grammes compris entre 50 et 1500 g/m².

11. Bande composite selon l'une quelconque des revendications précédentes, comprenant au moins une bande auxiliaire (5, 6) couplée à la masse (2) de fibres fondues-soufflées (3) dans laquelle le matériau particulaire (4) est dispersé.

12. Bande composite selon la revendication 11, dans laquelle la bande auxiliaire (5, 6) comprend une première bande (7) de tissu non tissé, la première bande de tissu non tissé ayant de préférence un poids en grammes compris entre 1 g/m² et 20 g/m².

13. Bande composite selon la revendication 11 ou 12, dans laquelle la bande auxiliaire (5, 6) comprend une seconde bande (8) de tissu non tissé ayant un poids en grammes compris entre 10 g/m² et 300 g/m².

14. Bande composite selon l'une quelconque des revendications 11 à 13, dans laquelle la bande auxiliaire (5, 6) et/ou la première bande (7) de tissu non tissé et/ou la seconde bande (8) de tissu non tissé et la masse (2) de fibres (3) fondues-soufflées sont liées sans matériaux adhésifs.

15. Article absorbant comprenant une pièce (103) d'une bande composite selon l'une quelconque des revendications précédentes.
